# EUROPEAN PATENT SPECIFICATION

(11) **EP 3 509 439 B1**
(45) Date of publication and mention of the grant of the patent: **08.10.2025**
(21) Application number: 17784333.1
(22) Date of filing: 08.09.2017
(51) Int. Cl.: A01K 67/65, A61K 35/63, A01K 67/67, A23L 33/00

(54) **METHODS OF PREPARING ARTHROPOD MEALS AND USES THEREOF**
VERFAHREN ZUR HERSTELLUNG VON ARTHROPODENSCHROTEN UND DEREN VERWENDUNGEN
PROCÉDÉS DE PRÉPARATION DES FARINES D'ARTHROPODE ET DE LEURS UTILISATIONS

(30) Priority: 08.09.2016 FI 20165670
(43) Date of publication of application: 17.07.2019
(73) Proprietor: Volare Oy, 28130 Pori (FI)
(72) Inventor: NORDLUND, Emilia, 02044 VTT (FI); HAKALA, Terhi, 02044 VTT (FI); ROMMI, Katariina, 02044 VTT (FI); SIPPONEN, Mika, 02044 VTT (FI); KAJALA, Ilkka, 02044 VTT (FI)
(74) Representative: Kolster Oy Ab
(86) International application number: PCT/FI2017/050637
(87) International publication number: WO 2018/046802

(56) References cited:
- WO-A1-2016/108036
- CN-A- 102 603 921
- DATABASE WPI Week 201311, Derwent World Patents Index; AN 2012-N53685, XP002775402

## Description

### FIELD OF THE INVENTION

The invention relates to the utilization of arthropods for preparing products, such as food products that meet consumer acceptance criteria, by technologically sound means. More specifically, the invention relates to a method of preparing valuable arthropod products, such as insect meals. The invention also relates to uses thereof. Furthermore, the invention relates to the use of defatted arthropod fractions for preparing said products.

### BACKGROUND OF THE INVENTION

Arthropods such as insects offer an excellent source of proteins with high nutritional value and balanced amino acid composition typical of animal proteins. There are also many other reasons that promote the nutritional use of insects, such as the need to find more sustainable protein sources and to improve food security. Insects can be reared efficiently independent of arable land and using low-cost fodder such as food waste. For instance, the feed conversion efficiency of crickets (*Acheta domesticus*) is fivefold compared to that of pigs (van Huis, 2013). Insects are consumed by estimated 2 billion people primarily in Asia, Africa, and South America. If the consumption of insects can be increased globally the growing world population may face less detrimental ecological changes.

However, especially Western consumers have shown prejudice over the consumption of insects. Therefore, attempts have been made to provide insect-based food ingredients that do not contain identifiable anatomical parts that will make insects more acceptable to consumers. For example, US 2014/295051 discloses an insect-based nutritional supplement comprising powderized insects. The manufacturing process involves cooking of insects in boiling liquid as well as oven-drying of the cooked insects in temperatures over 100°C resulting in denaturation of proteins. Moreover, the manufacturing process requires mixing of the insect powder with nutritional supplements, namely whey protein isolate and coconut, to optimize the amino acid profile of the mixture for human consumption.

Some efforts to isolate and characterize insect proteins have been based on the wet extraction methodology, which is industrially used in plant protein production. For instance, approximately 20% of protein could be solubilized by aqueous extraction of crickets and mealworms, the rest remaining in two wet solid fractions generated (Yi et al., 2013).

Also US 2015/0374005 discloses a wet extraction method of processing insects into nutrient streams, namely a fat-containing fraction, an aqueous protein fraction and/or a solid-containing fraction. However, wet fractionation requires energy-intensive water processing and results in large amounts of wastewater. Moreover, the method requires heating of insect pulp to a temperature of 70-100°C, which not only requires energy but also denatures insect proteins. Also, subjecting the heated pulp to a physical separation step, such as centrifuging, consumes energy.

WO 2016/108036 A1 discloses a method for the preparation of a composition involving killing of insects, pressing the insects to obtain a press cake, and drying and grinding the press cake. It is disclosed that the fat content of the composition is preferably between 5% and 20% by weight. The grinding step provides a particle size comprised between 300 µm and 1 mm.

Despite the attempts made, there is still a need in the art for sustainable and technologically sound insect or other arthropod-based products which contain proteins in natural, non-denatured from, as well as for methods of preparing the same.

### BRIEF DESCRIPTION OF THE INVENTION

An object of the invention is to provide arthropod-based products, such as insect meals for use as food or feed ingredients, which are technologically sound and meet the food and feed quality criteria. This object is achieved by a method and arrangements which are characterized by what is stated in the independent claims. Preferred embodiments of the invention are disclosed in the dependent claims.

The invention is, at least partly, based on studies aimed at exploring the potential of dry fractionation of defatted arthropods, such as crickets and mealworms, for providing effective processing methods to enable industrial utilization of arthropods in food and feed.

Defatting of arthropods, followed by dry-milling and fractionating the arthropod meal thus obtained provided protein-enriched fine and coarse fractions having deviating compositions and properties. For example, unexpected differences were detected in the flavour profiles of the fractions, the fine fractions being more intense in saltiness and in meat-like flavour.

The invention provides a method as claimed in claim 1. In one aspect, (not part of the invention) it is disclosed a method of preparing fine and coarse arthropod meals, characterized by comprising the steps of:
(a) extracting fat from arthropods, whereby a defatted arthropod fraction and one or more fat fractions are obtained,
(b) dry-milling the defatted arthropod fraction into arthropod meal having such a particle size that at least 90% of the particles are smaller than 120 µm, based on volume,
(c) dividing the thus obtained defatted arthropod fraction into a fine arthropod meal fraction and a coarse arthropod meal fraction by at least one operation selected from sieving and air classification, wherein said fine fraction has an average particle size below 40 µm, preferably below 15 µm, based on volume, and wherein said coarse fractions has an average particle size over 20 µm, preferably over 40 µm, based on volume.

In another aspect, (not part of the invention) it is disclosed an arthropod-based protein concentrate having a protein content of at least 40%, preferably at least 50%, more preferably at least 52%, based on amino acid determination, wherein cysteine represents at least 1.7%, preferably at least 1.9% of the total amino acids, lysine represents at least 6.2%, preferably at least 6.9% of the total amino acids, and methionine represents at least 1.4%, preferably at least 1.7% of the total amino acids.

In some embodiments, (not part of the invention) said concentrate may further comprise one or more characteristic selected from the group consisting of
a chitin content of less than 50%, preferably less than 42%, based on non-protein nitrogen;
a fat content of less than 5%, preferably less than 2.5%, more preferably less than 1%, based on dry matter; and
a volume-average particle size of less than 40 µm, preferably less than 15 µm.

In a further aspect, the disclosure (not part of the invention) provides an arthropod-based coarse meal having
a protein content of at least 45%, preferably at least 53%, more preferably at least 55%, based on amino acid determination;
a chitin content of less than 55%, preferably less than 47%, based on non-protein nitrogen;
a fat content of less than 5%, preferably less than 2.5%, more preferably less than 1%, based on dry matter; and
a volume-average particle size of over 20 µm, preferably over 40 µm.

In still further aspects, the disclosure (not part of the invention) provides use of a fine or coarse arthropod meal obtainable by the present method, as well as use of the present arthropod-based concentrate or the present arthropod-based coarse meal in foodstuffs, animal fees, pharmaceutical industry, and cosmetics.

In a still further aspect, the disclosure, (not part of the invention) provides use of defatted and dry-milled or ground arthropods for preparing arthropod meals by a mechanical dry method, namely air classification.

Some specific embodiments of the invention are set forth in the dependent claims. Other objects, details, embodiments, and advantages will become apparent from the following drawings, detailed description and examples.

### BRIEF DESCRIPTION OF THE DRAWINGS

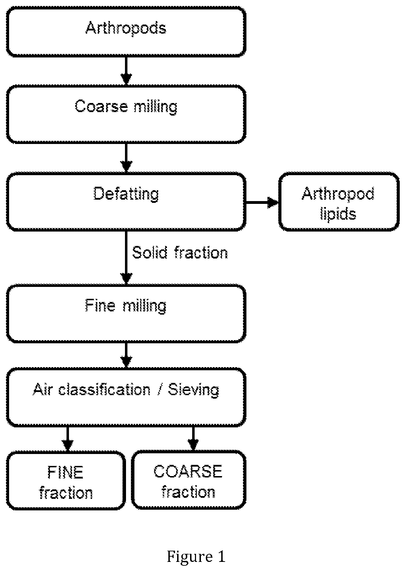

In the following the invention will be described in greater detail by means of preferred embodiments with reference to the attached drawings, in which
Figure 1 is a flowchart of the present method of providing fine and coarse arthropod meals.
Figure 2 shows distribution of lipid classes in crickets and mealworms before and after extraction with sc-CO₂. Phospholipids (PL), triglycerides (TG), diglycerides (DG), and free fatty acids (FFA) are shown.
Figure 3 shows the yields of fine (F) and coarse (C) fractions of sc-CO₂ extracted insects after fine milling and air classification at different classifier rotor speeds and airflow rates. Error bars shown for the 6000 rpm fractions from crickets indicate average deviation from the mean.
Figure 4 shows particle size distribution of fine (F) and coarse (C) fractions produced from sc-CO₂ extracted, fine-milled crickets and mealworms at 6000 rpm rotor speed in air classification.
Figure 5 shows a stained SDS-PAGE gel of proteins extracted from CO₂-defatted (1 and 4) insect meals and their fine (2 and 5) and coarse (3 and 6) fractions from air classification at 6000 rpm rotor speed. Lanes 1-3 are from mealworms and 4-6 from crickets.
Figure 6 shows crude protein contents of Wiley-milled and SC-CO₂ extracted insects as well as fine (F) and coarse (C) fractions after fine milling and air classification. Crude protein was calculated based on total nitrogen content of the samples using a conversion factor of N*6.25. Error bars indicate average deviation from the mean value of duplicate measurements.
Figure 7 is a visualization of chitin in air-classified (6000 rpm rotor speed) insect powders stained with Calcofluor: (a) fine fraction of crickets, (b) coarse fraction of crickets, (c) fine fraction of mealworms, (d) coarse fraction of mealworms.
Figure 8 shows the effect of pH on water solubility (solid line) and zeta-potential (dashed line) of proteins from crickets extracted with supercritical carbon dioxide. Zero zeta-potential levels are marked with horizontal lines.
Figure 9 demonstrates protein water solubility at their native pH of defatted insect meals (black bars) and their fine (gray bars) and coarse (white bars) fractions from air classification at 6000 rpm rotor speed.
Figure 10 demonstrates solubility in different solutions of mealworm and cricket meals obtained by coarse milling, extraction with supercritical carbon dioxide, and fine milling.
Figure 11 shows the results of sensory profiling (n = 2 × 10)of fine (F) and coarse (C) fractions of crickets and mealworms obtained by coarse milling, extraction with supercritical carbon dioxide, fine milling, and air classification at 6000 rpm rotor speed.

### DETAILED DESCRIPTION OF THE INVENTION

The invention relates to a method of preparing arthropod meal products by subjecting arthropods to defatting using methods such as supercritical extraction, typically with CO₂, and by treating the thus obtained defatted arthropod fraction by mechanical dry methods, such as milling, sieving and air classification.

As used herein, the term "arthropod" refers to any species of the phylum *Arthropoda* in any developmental stage, including larvae, pupae, and adult arthropods. Arthropods are characterized by exoskeletons mainly composed of chitin, and include insects, arachnids, myriapods, and crustaceans. For use in the present invention and its various embodiments, insect species are preferred.

As used herein, the term "insect" refers to any species of the class *Insecta* in any developmental stage. Preferred insects include, but are not limited to, edible insects selected from crickets (suborder *Ensifera,* family *Gryllidae*), grasshoppers (suborder *Caelifera*) such as locusts (family *Acrididae*) and katydids (family *Tettigoniidae*), beetles (order *Coleoptera*), and ants (family *Formicidae*). More specifically, preferred beetles include, but are not limited to, mealworm beetle (*Tenebrio molitor*), especially in the larval form, i.e. as mealworms. Preferred crickets include, but are not limited to, a house cricket (*Acheta domesticus*). For use in the present invention, insects may be harvested from nature or, preferably, obtained by insect rearing in controlled conditions.

In some embodiments, preferred arthropods include insects and crustaceans in any developmental stage, including larvae, pupae, and adults.

In the present invention, euthanized arthropods of a single or multiple species are used as a starting material, preferably in dried form. Typical euthanizing methods include freezing, or suffocating with CO₂ or N₂. If a freezing method is used, it is convenient to dry the arthropods by freeze-drying. However, freeze drying may be applied regardless of the euthanizing method. Other non-limiting examples of suitable, optional, drying methods include air drying, and washing with ethanol. It may be advantageous to carry out the air drying in a temperature below 60 °C to avoid denaturation of arthropod proteins. Without drying the moisture content of arthropods such as crickets and mealworms varies typically between about 65% and about 70%. In some embodiments, drying reduces the moisture of the arthropods down to 0-40%, preferably to less than 10%, even more preferably to less than 5%. Reduced moisture of the starting material may improve the performance of or hasten the present method, especially the defatting step by supercritical extraction. However, in some other embodiments, no pre-drying of the starting material is to be carried out.

Alternatively or additionally, the arthropods may be subjected to a short heat treatment for instance at temperatures not exceeding 140°C, such as at temperatures ranging between 70°C and 100°C, for a duration of time not exceeding 15 min, such as for 1 min to 10 min, to kill any micro-organisms or inactivate enzymes prior to carrying out the present method. Such an optional heat pretreatment may be employed also to improve the shelf life and overall quality of the present arthropod products. However, too long or too harsh a heat treatment may result in denaturation of arthropod proteins, or otherwise impair technical features of the arthropod proteins, and is thus to be avoided. To be more specific, denaturation may complicate subsequent milling and classification operations, for example. Thus, a preferred heat treatment does not result in any measurable changes in the arthropod proteins.

Further non-limiting alternative or additional pre-treatments include removing of anatomical parts such as legs and wings, cutting, crushing, and/or grinding. Such pre-treatments are fully optional but may be employed to improve the efficiency of a subsequent defatting step, especially by enhancing fat extraction or shortening the duration thereof. In some embodiments, arthropods are pre-treated by grinding to pass a 3 mm sieve without removing any anatomical parts. Suitable sieve opening size (diameter) may be also smaller than or larger than 3 mm, for instance 1-3 mm or 3-10 mm.

In accordance with the above, there is disclosed a method of preparing arthropod products, the method comprising:
(a') subjecting arthropods to one or more pre-treatments selected from the group consisting of drying, heat treating, removing selected anatomical parts, cutting, crushing, and grinding,
(a) extracting fat from pre-treated arthropods, whereby a defatted arthropod fraction and one or more fat fractions are obtained,
(b) dry-milling the defatted arthropod fraction into arthropod meal having such a particle size that at least 90% of the particles are smaller than 150 µm, preferably smaller than 120 µm, based on volume,
(c) dividing the thus obtained defatted arthropod fraction into a fine fraction and a coarse fraction by at least one operation selected from sieving and air classification, wherein said fine fraction has an average particle size below 40 µm, preferably below 15 µm, based on volume, and wherein said coarse fractions has an average particle size over 20 µm, preferably over 40 µm based on volume.

Some other embodiments, which involve no pre-treatments, relate to a method of preparing arthropod products, the method comprising:
(a) extracting fat from arthropods, whereby a defatted arthropod fraction and one or more fat fractions are obtained,
(b) dry-milling the defatted arthropod fraction into arthropod meal having such a particle size that at least 90% of the particles are smaller than 150 µm, preferably smaller than 120 µm, based on volume,
(c) dividing the thus obtained defatted arthropod fraction into a fine fraction and a coarse fraction by at least one operation selected from sieving and air classification, wherein said fine fraction has an average particle size below 40 µm, preferably below 15 µm, based on volume, and wherein said coarse fractions has an average particle size over 20 µm, preferably over 40 µm based on volume.

The present method comprises an extraction step (a) wherein fat is removed from arthropods by any defatting technique available in the art, provided that the technique does not result in substantial denaturation of arthropod proteins or otherwise impair functional or technical characteristics thereof. Step (a) yields a defatted arthropod fraction and one or more fat fractions. Said one or more fat fractions which consist mainly of triglycerides contain about 70% or more of the fats of the arthropods. If desired, said one or more fat fractions may be recovered. The fat content of the defatted arthropod fraction is typically between about 3% and about 5%, but in some embodiments preferably less than about 2.5%, more preferably less than about 1%. Residual fats in the defatted arthropod fraction comprise mainly phospholipids.

Preferred defatting techniques include conventional solvent extraction techniques, and extraction with a fluid in a supercritical state, the fluid typically being carbon dioxide. Non-limiting examples of suitable solvents for solvent extraction include non-polar solvents such as hydrocarbons (e.g. hexane or heptane), chlorinated hydrocarbons (e.g. dichloromethane or chloroform), ethers (e.g. diethyl ether or methyl tert-butyl ether, MTBE), and mixtures thereof. Those skilled in the art can easily select an appropriate solvent or solvent mixture to be used depending on the desired outcome of the defatting step. Solvent extraction may be carried out as generally known in the art using batch solvent extraction, semi-continuous solvent extraction (e.g. Soxhlet), or continuous solvent extraction (e.g. Goldfish), for example. In some embodiments, solvent extraction may be repeated two or more times using the same or different solvents and/or extraction techniques in order to improve fat removal.

Being a more gentle treatment, extraction with a fluid in a supercritical state is in some embodiments a preferred defatting technique. In some even more preferred embodiments, said supercritical fluid is supercritical carbon dioxide. In some further embodiments, said supercritical fluid extraction may be carried out with a combination of carbon dioxide and a C₁-C₄ alcohol, preferably ethanol. In such cases, the proportion of ethanol may be 8 to 10%, for example. The use of ethanol together with carbon dioxide improves the separation of polar lipids, such as phospholipids and glycolipids together with triglycerides. In addition, ethanol as a co-solvent enhances the efficiency of fat removal from moisturecontaining raw materials. When ethanol is to be employed as a co-solvent, defatting may be performed either in two steps, first by performing the extraction with carbon dioxide and then with a combination of carbon dioxide and ethanol, or preferably in one step with a combination of carbon dioxide and ethanol. Any of these embodiments may also be carried out by replacing ethanol, either in full or in part, with another C₁-C₄ alcohol, namely methanol, propanol, isopropanol, 1-butanol, or isobutanol, or any mixture thereof.

Supercritical extraction is carried out by using temperature and pressure conditions wherein the extraction fluid used is in a supercritical state. The extraction temperature is typically within the range 32 to 90°C, for instance 40°C, and the extraction pressure is typically within the range 120 to 600 bar, for instance 300 bar. The flow rate is typically between 11 L/h and 16 L/h, for instance 12.5 L/h. The extraction time may vary depending on the batch size between 1 h and 4 h, and it may be 80 to 140 min, for example. Under these conditions, arthropod proteins become significantly less denatured than generally in defatting by organic solvents which typically involves processing steps at temperatures above 60°C. This is a significant advantage, since it facilitates the separation of the fractions in the air classification and sieving subsequent to the milling.

Supercritical extraction not only removes fat, but also removes water. Preferably, the arthropod fraction to be subjected to subsequent mechanical dry methods contains less than 10%, preferably less than 5% of moisture. This feature prevents or reduces unwanted aggregation that would impair fractionation of dry-milled defatted arthropod fractions.

The defatted arthropod fraction obtained as set forth above is fractioned further by mechanical dry methods, such as dry milling, sieving and air classification into different functionally valuable products. In contrast to wet fractionation, dry fractionation avoids energy-intensive water processing and the generation of a large amount of wastewater.

The fractionating of defatted arthropod fraction comprises dry milling, for example by impact milling (step b of the present method). In the impact milling, a pin mill, a hammer mill, or mills provided with grinding discs or mills provided with a sieve may be used, for example. The milling may be performed in one or more steps, and it may or may not comprise, either before or after said milling, a step wherein anatomical parts, such as legs or wings, are removed. The removal of the anatomical parts may be performed with the aid of a fluidized bed, for example. The milling provides an arthropod meal having such a particle size that at least 90% of the particles are smaller than 150 µm, preferably smaller than120 µm calculated on the basis of the volume.

In step (c) of the present method, the arthropod meal obtained by step b) is divided into a fine fraction and into a coarse fraction. As set forth above, said fine fraction has an average particle size below 40 µm, preferably below 15 µm, calculated on the basis of the volume. In other words, said fine fraction has such a particle size distribution that particles having a diameter smaller than 80 µm, preferably smaller than 40 µm, constitute at least 90% of the particles in the fraction. Said coarse fraction, in turn, has an average particle size over 20 µm, preferably over 40 µm, calculated on the basis of the volume. On the other hand, said coarse fraction has such a particle size distribution that particles having a diameter smaller than 200 µm constitute at least 90% of the particles in the fraction.

In step (c) of the present method, said division is carried out by using one or more operations selected from sieving, air classification, and any combination thereof. In air classification, the process parameters are preferably the following: rotation speed of the classifier wheel of the air classification device is about 20 to 100% of the maximum rotation speed of the classifier, and the air flow of the air classification device is 40 to 100% of the maximum air flow of the classifier. Typically, the rotation speed of the classification wheel may vary between 3000 rpm and 22000 rpm, and be more specifically 3000 rpm, 4000 rpm, 5000 rpm, or 6000 rpm, for example.

Essential components comprised by an air classification device that may to be employed in the present method are a classifier chamber, a cyclone, a collector vessel and a filter following the cyclone. A coarse fraction of the classification is recovered from the collector vessel, a fine fraction from the cyclone and a finest fraction from the filters. Air classification devices are readily available in the art.

In some embodiments, classification of the dry-milled defatted arthropod fraction is carried out by sieving. The sieve opening sizes used in the sieving are typically in the range of 20 to 250 µm, preferably 25 to 100 µm. Sieving may be performed for instance as an air jet sieving.

The fine fraction and/or the coarse fraction obtained in step (c) of the present method may be further divided by sieving, air classification, or any combination thereof into one or more further fine fractions and/or one or more further coarse fractions to obtain arthropod meals having different characteristics. Said further dividing may involve dry-milling of the first coarse fraction, for example. Accordingly, step (c) of the present method may in some embodiments include a plurality of steps, whereby the coarse fraction of the first sieving or air classification is milled and sieved or air-classified again.

In one such embodiment , step (c) includes additional milling, allowing the method to be carried out in the following manner: the arthropod meal obtained from step (b) is sieved with a sieve having a sieve opening size of 25 to 100 µm, and the coarse fraction remained on the sieve is recovered. This coarse fraction is re-milled, for instance by impact milling, and the thus obtained powder is divided by sieving (sieve opening size 20 to 150 µm, preferably 25 to 50 µm) or by air classification into a fine fraction and a coarse fraction, yielding the fine fraction and the coarse fraction according to step (c) of the present method.

A fine fraction obtainable by the present method may be referred to as an arthropod-based protein concentrate or an arthropod-based fine meal having a protein content of at least 40%, preferably at least 50%, more preferably at least 52%, based on amino acid determination, wherein cysteine represents at least 1.7%, preferably at least 1.9% of the total amino acids, lysine represents at least 6.2%, preferably at least 6.9% of the total amino acids, and methionine represents at least 1.4%, preferably at least 1.7% of the total amino acids. In some embodiments, said concentrate or fine meal may comprise one or more further characteristics selected from the group consisting of
a chitin content of less than 50%, preferably less than 42%, based on non-protein nitrogen;
a fat content of less than 5%, preferably less than 2.5%, more preferably less than 1%, based on dry matter and
a volume-average particle size of less than 40 µm, preferably less than 15 µm.

The coarse fraction obtainable by the present method may be referred to as an arthropod-based coarse meal having a protein content of at least 45%, preferably at least 53%, more preferably at least 55%, based on amino acid determination;
a chitin content of less than 55%, preferably less than 47%, based on non-protein nitrogen;
a fat content of less than 5%, preferably less than 2.5%, more preferably less than 1%, based on dry matter and
a volume-average particle size of over 20 µm, preferably over 40 µm.

The disclosure further relates to fine and coarse arthropod meals having one or more of the characteristics set forth above and obtained by the present method or any embodiments thereof, as well as to fine and coarse arthropod meals having these characteristics regardless of the method used for their preparation.

The present low-fat arthropod meals are expected to have improved shelf lives since gustatory detriments caused by fat hydrolysis or oxidation have less tendency to form since the defatting step has removed easily accessible lipids. In addition, they are easy to dose and do not cause clumping in the air classification equipment in contrast to conventional, fat-containing meals prepared from arthropods. They also easily disperse in water and in some other solutions, thereby improving their suitability for food processing. The present products differ from products prepared by wet extraction methods in that the proteins remain in natural, non-denatured form. In the food industry, the products are usable for various purposes, such as fibre supplements, expansion supplements, viscosity supplements, dispersion supplements and protein supplements.

The present fine fraction has improved sensory quality (flavour and mouthfeel) as compared with that of the coarse fraction. The fine fractions had significantly less coarse mouthfeel than the coarse fractions which are expected to improve their applicability for a broad variety of food products. Additionally, the fine fractions exhibited saltier and more intense meat-like flavour with stronger flavour intensity compared to those of the coarse fractions. Furthermore, the fine fractions contained essential amino acids in amounts which exceed the Food and Agriculture Origanization's (FAO) dietary protein quality criteria, and were enriched with essential amino acids cysteine, lysine, and methionine, in particular. Accordingly, the present fine fractions find multiple uses in food and feed industry including, but not limited to, their use as protein supplements, nurtaceuticals, food additives, feed additives, and flavour intensifiers.

In the present invention, the chitin particles of the arthropod exoskeletons become detached during milling, from where the largest chitin particles were separated by air classification into the coarse fraction. The coarse fraction, containing typically more chitin than the fine fraction, may be used for arthropod products where structure formation or high fiber content is desirable, for example. Accordingly, the present coarse faction may be applied as fiber additives, abrasives, nutraceuticals, pharmaceuticals, nutrient supplements, food additives, or feed additives. It may also be used for making chitosan, or a raw material for making plastics and films.

Furthermore, the invention also relates to the use of defatted arthropods, especially arthropods defatted with supercritical CO₂ extraction, for preparing arthropod meals with varying characteristics by mechanical dry methods selected from dry milling, sieving and air classification. The fat content of the defatted arthropod fraction is typically between 3% and 5%, but may be even as low as 2.5% or less, more preferably even less than about 1%.

### EXAMPLES

### Example 1. Supercritical extraction and lipid analyses

Freeze-dried crickets and mealworms harvested at adult stage and larval stage, respectively, were purchased from a commercial provider. The insects were ground to pass a 3 mm sieve in a Wiley mill for defatting.

Extraction of ground insects in two batches of 250 g with supercritical carbon dioxide (sc-CO₂) was conducted under 300 bar pressure in a Multi-Use SFE Plant with a pressure vessel of 10 L (Chematur Ecoplanning, Rauma, Finland). The extraction time was 80-140 min and the flow rate of sc-CO₂ was 12.5 L/h. The temperature was maintained at 40 °C during the extraction and 46 °C in the separation chamber. The yields of lipid fractions and extraction residues were recorded for the calculation of mass balances.

The insect materials were analysed for their crude protein and lipid contents before and after extraction with sc-CO₂. Nitrogen was analysed using a Kjeldahl autoanalyzer (Foss Tacator Ab, Höganäs, Sweden), and crude protein was calculated as N × 6.25 according to the method 46-11A (AACC, 2000). Lipid analyses incorporated the analysis of total fatty acids and major lipid classes of triglycerides (TG), diglycerides (DG), phospholipids (PL), and free fatty acids (FFA). The procedures used in direct saponification, extraction, methylation, and gas chromatography of fatty acid methyl esters as well as the separation of major lipid classes in thin layer chromatography are described in detail elsewhere (Sipponen et al., 2016).

The results in Table 1 show that Crickets and mealworms contained 24.3% and 35.6% total lipids determined as total fatty acid methyl esters on the basis of dry weight. These results accord well with the literature values of 22.1% and 34.4% calculated for crickets and mealworms, respectively (Finke, 2002; Nowak et al., 2016). The defatting enriched crude protein in the insect meals (Table 1). The changes in crude protein contents were from 64% to 79% (crickets) and 52% to 73% (mealworms). The corresponding lipid removal was 79% and 74% from crickets and mealworms, leaving 4.8% and 3.5% lipids in the extraction residues. Degree of unsaturation (DUS), which describes the proportion of double bonds in the lipids (amount of double bonds/total amount of FAME) was higher in the extraction residues. The lipids of crickets and mealworms had DUS-value 1.09 and 1.17, respectively. Instead, after sc-CO₂-extraction the corresponding values were 1.33 and 1.34. A closer look at the distribution of fatty acids revealed enrichment of stearic acid (C18:0) and especially linoleic acid (C18:2) due to the extraction. To better understand this fractionation of fatty acids, lipid classes were determined from the same materials.

**Table 1. Lipid content (as total fatty acids methyl esters, FAME) and fatty acid distribution in crickets and mealworms before and after sc-CO2 extraction**

| Material | Crude protein* | Total FAME | Fatty acid distribution (% of total FAME) | | | | | | |
|---|---|---|---|---|---|---|---|---|---|
| | (% db) | | C14:0 | C16:0 | C16:1 | C18:0 | C18:1 | C18:2 | C18:3 |
| Crickets | 63.8 | 24.3 | 2.1 | 25.8 | 0.5 | 6.7 | 23.7 | 39.3 | 1.9 |
| sc-CO₂ extr. crickets | 78.9 | 4.8 | 0.3 | 15.0 | 0.5 | 13.3 | 12.7 | 57.2 | 1.0 |
| Mealworms | 51.9 | 35.6 | 3.3 | 16.9 | 0.8 | 2.1 | 39.9 | 34.5 | 2.4 |
| sc-CO₂ extr. mealworms | 73.2 | 3.5 | 0.3 | 12.0 | 0.3 | 11.3 | 21.4 | 53.5 | 1.0 |

| | | | | | | | | | |
|---|---|---|---|---|---|---|---|---|---|
| *calculated based on total nitrogen content using a conversion factor of N*6.25. | | | | | | | | | |

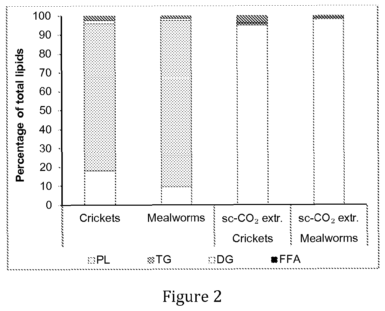

Triglycerides (TGs) comprised 78% and 88% of total lipids of crickets and mealworms before defatting. After extraction with sc-CO₂, most of the TGs were removed and the residual lipids comprised almost exclusively of phospholipids (Figure 2).

### Example 2. Fine milling and air classification

Defatted insects obtained as described in Example 1 were milled twice at 17800 rpm with a Hosokawa-Alpine multi-purpose mill UPZ-lb (Hosokawa Alpine, Augsburg, Germany) assembled with a pin disc milling unit. The finely ground insects were subjected to air classification using a 50 ATP classifier (Hosokawa Alpine, Augsburg, Germany) and a Minisplit Classifier (British Rema Manufacturing Company Ltd., UK) to determine fractionation and solubility of proteins, as well as to produce fractions with contrastive particle size distributions for sensory analysis.

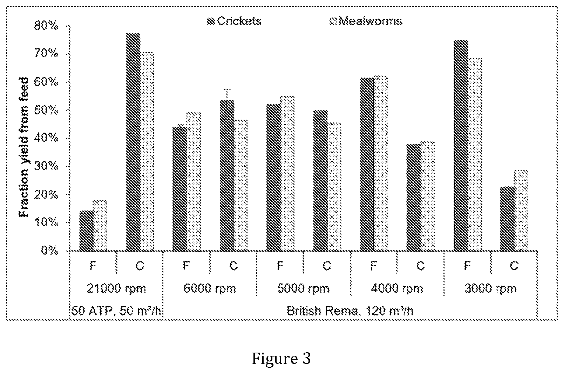

The yield of fine (F) and coarse (C) fractions was calculated as per the amount weighed to air classification (100 g or 50 g). The yield of fine fractions increased linearly (R2>0.99) when the rotor speed decreased from 6000 to 3000 rpm (Figure 3). The lowest yields of the fine fraction were 14% for crickets and 18% for mealworms and obtained at the highest rotor speed of 21000 rpm. The fine and coarse fractions were obtained at roughly equal yields at rotor speed of 6000 rpm, and were selected for further particle size distribution and sensory analysis tests because of their distinct visible appearances.

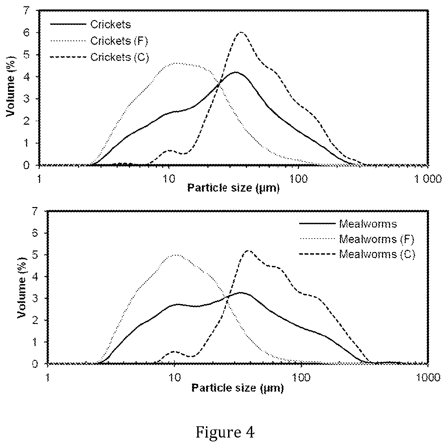

The particle size distributions of defatted insect meals and their selected fine and coarse fractions from air classification were determined by laser diffraction (Figure 4) using a Beckman Coulter LS 230 (Beckman Coulter Inc., CA, USA) laser diffraction particle size analyser with the liquid module and degassed deionized water as a carrier fluid. Refractive index values of 1.333 and 1.456 were used for degassed deionized water and insect proteins, respectively.

Insect meals from crickets and mealworms showed broad particle size distributions from 2.5 to 350 µm with corresponding mean particle sizes of 26.7 ± 0.1 µm and 27.1 ± 0.3 µm. These similar values reflect that the milling treatment after defatting was equally efficient with either of the insect meals. Air classification had a different impact on the fractionation of the insect meals. The fine fraction from crickets showed a broad distribution in the 2.5 to 180 µm range, whereas mealworms showed a narrow distribution from 55 to 90 µm. Despite these differences, the mean particle sizes of the fine fractions were almost similar, 13.9 for crickets and 12.2 for mealworms. The D90 values of the fine fractions from crickets (36.3 µm) and mealworms (30.5 µm) were used to calculate the constants C from Eq. 1. For crickets and mealworms values of 19880 and 16705 were obtained for the constant C. Using these values, the calculated D90 values of the fine fractions from air classification at 21000 rpm were 7 µm for crickets and 6 µm for mealworms. Air classification can therefore be used to fractions with wellsuited coarseness to the desired application. The coarse fractions showed distributions that resulted from exclusion of the fine particles from original defatted insect meals.

### Example 3. Fine milling and sieving

Mealworm material defatted by extraction with SC-CO₂ as described in Example 1 was ground twice with pin mill at 17800 rpm and subjected to sieving with a Retsch AS 200 equipment using a combination of sieves with following opening sizes (from top down): 315 µm, 250 µm, 125 µm, 50 µm, and 25 µm.

First, 10.4 g of mealworm meal was weighed to sieving at the initial amplitude (unit: mm/"g") of 1.5 for 10 s and then at the amplitude of 0.20 for 15 min. The produced fractions in the sieves were weighed, and the results are shown in Table 2 below.

**Table 2.**

| Sieve opening size (µm) | Empty sieve (g) | Sieve + meal worm meal fraction (g) | Ratio of material on the sieve to the initial amount of mealworm meal (g/g) |
|---|---|---|---|
| collection plate | 361.3 | 361.3 | 0.00 |
| 315 | 262.9 | 262.9 | 0.00 |
| 250 | 247.4 | 247.5 | 0.01 |
| 125 | 275.8 | 280.4 | 0.44 |
| 50 | 366.9 | 371.1 | 0.40 |
| 25 | 375.3 | 376.6 | 0.13 |

Next, the sieving time was prolonged. Sequential sieving and weighing of the fractions was conducted to follow the time-course of sieving. The results are shown in Table 3 below.

**Table 3.**

| Sieving time (min) at amplitude | | | | | | |
|---|---|---|---|---|---|---|
| | | 15 min at 0.5 ampl. | 15 min at 0.5 ampl. + 15 min at 1.0 ampl. | 15 min at 0.5 ampl. + 30 min at 1.0 ampl. | 15 min at 0.5 ampl. + 60 min at 1.0 ampl. | |
| Sieve opening size (µm) | Empty sieve (g) | Sieve + mealworm meal fraction (g) | | | | Final ratio of material on the sieve to the initial amount of mealworm meal (g/g) |
| collection plate | 361.3 | 361.3 | 361.3 | 361.3 | 361.3 | 0.00 |
| 315 | 262.9 | 263.0 | 263.0 | 262.9 | 263.0 | 0.01 |
| 250 | 247.4 | 247.5 | 247.5 | 247.5 | 247.3 | -0.01 |
| 125 | 275.8 | 279.8 | 277.0 | 276.6 | 276.4 | 0.06 |
| 50 | 366.9 | 371.5 | 371.4 | 370.9 | 370.9 | 0.42 |
| 25 | 375.3 | 376.1 | 378.8 | 379.6 | 379.8 | 0.47 |

The results show that by prolonged sieving duration a higher proportion of the fine fraction that passed the 50 µm sieve but not the 25 µm sieve was obtained. After a total of 90 min of sieving at amplitude from 0.5 to 1.0, the ratio of material collected on the 25 µm sieve to the initial amount of mealworm meal was 0.47 compared to 0.13 when the sieving was conducted for a shorter duration (Table 2). The fractionation of material according to the particle size can therefore be controlled by the sieving time.

### Example 4. Protein and chitin analyses

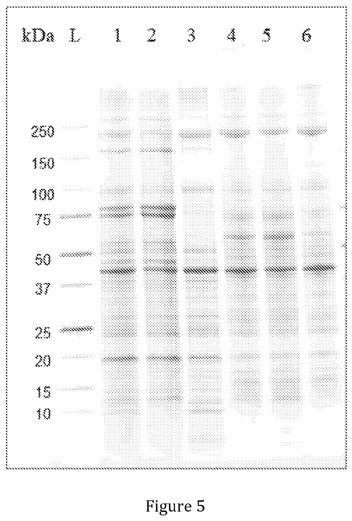

Defatted insect meals obtained as described in Example 1 and their fine and coarse fractions obtained as described in Example 2 were subjected to protein extraction and subsequent electrophoretic analyses (Figure 5). The extraction was carried out in in 2% SDS, 6 M urea, 0.1 M DTT. Extracts were treated with sample buffer, and loaded in 4-20% Criterion^{™} TGX Stain-Free^{™} Protein Gels (Bio-Rad) with Precicion plus markers (Bio-Rad). The gels were stained with colloidal coomassie G-250 (PageBlue Protein Staining Solution, Thermo Scientific, Rockford, IL) and imaged with an EZ Imaging system (Bio-Rad). Quantification of protein bands across individual lanes was performed by image analysis using Im-ageJ (Windows version). All samples were diluted in the same manner to enable observing differences in quantities and composition between fractions.

The main bands were present also in gels run under non-reducing conditions, indicating the absence of disulphide-bound subunit structures (data not shown). The most intense bands had relative molecular weights (MWR) of 19 kDa, 40 kDa, and a double band at 75 and 77 kDa (lane 1; Figure 5), and 40 and 60 kDa in mealworms (lane 4). Dry fractionation of mealworms enriched two bands with MWR of 75 and 77 kDa, two fainter bands with MWR of 55 kDa and a bands >150 kDa in the fine fraction (lanes 1-2). Bands visible at 40 kDa (possibly actin) and just below the 250 kDa marker (possibly myosin) were somewhat enriched in the coarse fraction (lane 3).These proteins are possibly actin and myosin, respectively (van Straaten et al., 1999). Fewer differences were observed in cricket samples: only a band at 60 kDa was slightly enriched in the fine fraction and faint high molecular weight bands (>250 kDa) were slightly enriched in the coarse fraction. The latter may be kettin and other flight- and leg muscle proteins (Bullard and Leonard, 1996; van Straaten et al., 1999). This is in accordance with the microscopic observation of fragments of these anatomical parts in the coarse fractions.

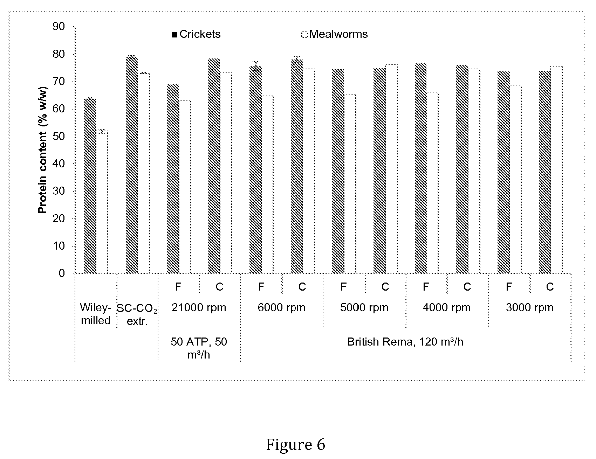

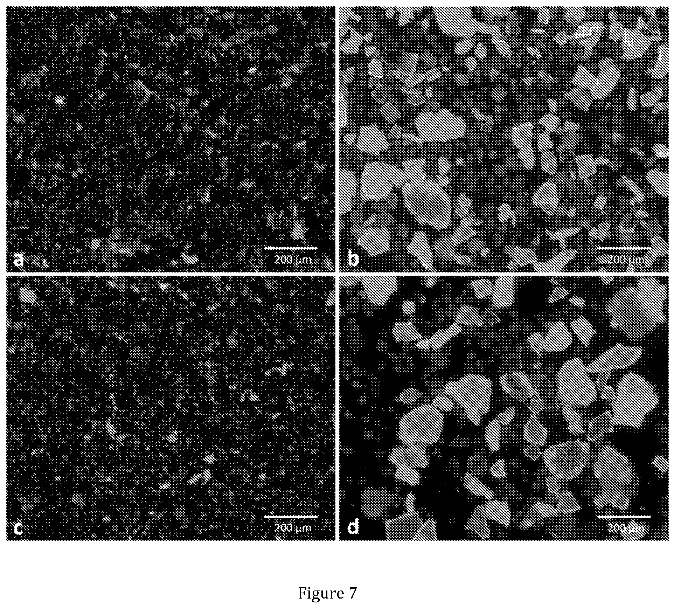

Analysis of crude protein by nitrogen assessment with a Kjeldahl autoanalyzer (Foss Tacator Ab, Höganäs, Sweden) and by subsequent crude protein calculations using the equation N × 6.25 according to the method 46-11A (AACC, 2000) did not reveal fractionation as a result of air classification (Figure 6). However, it is known that crickets and mealworms contain 5-10% chitin mainly bound in the exoskeleton. Therefore, fractionation of chitin in the durable shell layers into the coarse fractions could be erroneously interpreted as crude protein in the Kjeldahl total nitrogen determination. To elucidate this, staining of chitin in the fine and coarse fractions with Calcofluor was carried out. For this, 3-4 mg of sample was stained with 80 µl of aqueous 0.01% (w/v) Calcofluor White (Fluorescent brightener 28, Aldrich, Germany) on objective glass. After careful mixing, preparate was covered with a cover slip and sealed. The samples were examined under exciting light (excitation, 390-420 nm; emission, >450 nm) with a Zeiss AxioImager M.2 microscope (Carl Zeiss GmbH, Göttingen, Germany). Micrographs were obtained using a Zeiss Axiocam 506 CCD colour camera (Zeiss) and the Zen imaging software (Zeiss). Representative images were chosen for Figure 7.

Fluorescence microscopy showed clear differences between fine and coarse fractions in size of the particles as well as in the chitin content (Figure 7). In line with the particle size distributions of these fractions, fine fractions consisted of particles with notably smaller size in comparison to coarse fractions. The particles in the fine fractions had some variability in shape (Figure 7, panels a and c). The fine fraction of crickets contained both elongated and more roundish or rectangular particles, while the fine fraction of mealworms consisted mostly of rectangular kind of particles. Both fine fractions had relatively low stainability with Calcofluor in general, although some chitin-rich structures with bright fluorescence were observed as well. Coarse fractions of both crickets and mealworms consisted of angular particles of which the largest ones were brightly stained with Calcofluor indicating high chitin content (Figure 7, panels b and d). Also some of the smaller particles were stainable with Calcofluor, and the overall stainability of the coarse fractions was clearly higher than that of the fine fractions.

On the basis of the Calcofluor staining, chitin content in the coarse fractions was significantly higher than in the fine fractions. Chitin is known as the component responsible for the high durability of the insect cuticle (Vincent and Wegst, 2004). Durability of chitin-rich structures was proved also in this study as the chitin-rich particles composed the majority of the coarse fraction with higher particle size. Moreover, despite 90 min ball milling at 20 s⁻¹ frequency, the particle sizes of the coarse fractions of crickets and mealworms could not be reduced to the level achieved in the fine fractions from air classification.

Amino acid profiles of defatted insect meals and fine and coarse fractions thereof were analysed by standard methods known in the art consisting of acid hydrolysis and liquid chromatography. The analysis excluded determination of tryptophan. The results show clear enrichment of essential amino acids cysteine, lysine and methionine into the fine fractions (Table 4).

**Table 4. Amino acid compositions of insect fractions**

| | Amino acids as % of total protein | | | | | |
|---|---|---|---|---|---|---|
| | Defatted, milled crickets | Defatted, milled crickets, 6000 rpm FINE | Defatted, milled crickets, 6000 rpm COARSE | Defatted, milled mealworms | Defatted, milled mealworms, 6000 rpm FINE | Defatted, milled mealworms, 6000 rpm COARSE |
| Histidine | 2.8 | 2.8 | 2.8 | 3.6 | 3.4 | 3.8 |
| Serine | 5.0 | 5.1 | 5.0 | 5.6 | 5.2 | 5.8 |
| Arginine | 7.9 | 8.0 | 7.3 | 5.9 | 6.2 | 5.8 |
| Glycine | 5.2 | 4.9 | 5.5 | 5.1 | 4.2 | 5.5 |
| Aspartic acid | 8.9 | 9.6 | 8.1 | 9.2 | 10.3 | 8.2 |
| Glutamic acid | 13.5 | 14.5 | 12.4 | 13.5 | 15.2 | 12.4 |
| Threonine | 4.1 | 4.3 | 3.8 | 4.3 | 4.4 | 4.1 |
| Alanine | 8.9 | 6.8 | 11.0 | 7.0 | 4.7 | 8.7 |
| Proline | 6.2 | 5.4 | 6.9 | 7.2 | 6.4 | 7.5 |
| Cysteine | 1.8 | 2.0 | 1.7 | 1.7 | 1.9 | 1.6 |
| Lysine | 6.6 | 7.2 | 5.8 | 6.2 | 6.9 | 5.9 |
| Tyrosine | 5.3 | 4.7 | 6.2 | 6.3 | 5.9 | 6.3 |
| Methionine | 1.7 | 2.0 | 1.4 | 1.4 | 1.7 | 1.3 |
| Valine | 5.8 | 5.5 | 6.4 | 6.2 | 5.4 | 6.5 |
| Isoleucine | 4.5 | 4.6 | 4.5 | 4.8 | 5.2 | 4.8 |
| Leucine | 8.1 | 8.3 | 8.0 | 8.0 | 8.6 | 8.2 |
| Phenylalanine | 3.8 | 4.3 | 3.2 | 3.9 | 4.5 | 3.5 |

Amino acid compositions and total nitrogen content of the insect fractions were used to calculate protein and chitin contents of the insect fractions (Table 5). Nitrogen not originating from amino acids was considered to originate from chitin. Chitin content was estimated based on approximate nitrogen contents of 7,87% (crickets) and 5,25% (mealworms) in chitin, as calculated from the data of Finke et al., 2007. It is generally known that amino acid-based protein calculations provide underestimated protein contents, for example owing to losses in acid hydrolysis and tryptophan content not being determined. Therefore, the true protein contents of the insect fractions are somewhat higher than those obtained through amino acid calculations. This may result in overestimation of non-protein nitrogen and, thus, in overestimation of the chitin content. This explains why the calculated sum of the protein and chitin contents of the factions exceeds 100% slightly.

**Table 5. Protein and chitin contents of insect fractions**

| Sample | Total protein^{a} % | Total N % | Protein N % | Non-protein N % | Estimated chitin ^{b} % |
|---|---|---|---|---|---|
| Defatted, milled crickets | 54.6 | 12.6 | 8.7 | 3.9 | 49.4 |
| Defatted, milled crickets, 6000 rpm FINE | 55.5 | 12.1. | 8.9 | 3.2 | 41.2 |
| Defatted, milled crickets, 6000 rpm COARSE | 55.1 | 12.5 | 8.8 | 3.7 | 46.7 |
| Defatted, milled mealworms | 57.8 | 11.7 | 9.2 | 2.5 | 47.1 |
| Defatted, milled mealworms, rpm 6000 FINE | 51.7 | 10.4 | 8.3 | 2.1 | 39.9 |
| Defatted, milled mealworms, rpm 6000 COARSE | 60.8 | 11.9 | 9.7 | 2.2 | 42.2 |

| | | | | | |
|---|---|---|---|---|---|
| ^{a} calculated on the basis of amino acid composition (excluding tryptophan) ^{b} calculated on the basis of nitrogen contents of 7.87% (crickets) and 5.25 (mealworms) in chitin. All non-protein nitrogen is assumed to originate from chitin. | | | | | |

### Example 5. Protein solubility and extractability

The solubility and zeta-potential of insect proteins were analysed after the sc-CO₂ extraction carried out as described in Example 1. To this end, samples (2 %) were dispersed in water and the pH was adjusted to 9. After stirring for 2 h, aliquots were taken and adjusted to pH 3-9 using HCl and NaOH. pH values were readjusted over a period of 2 h, and samples volumes were adjusted to a final concentration of 1%. Samples were centrifuged at 3000 g x 10 min and the protein contents of supernatants were determined against a BSA standard curve using a DC kit (Bio-Rad). ζ-potentials of the supernatants were determined using a Zetasizer Nano ZS (Malvern Instruments Ltd, Malvern, UK).

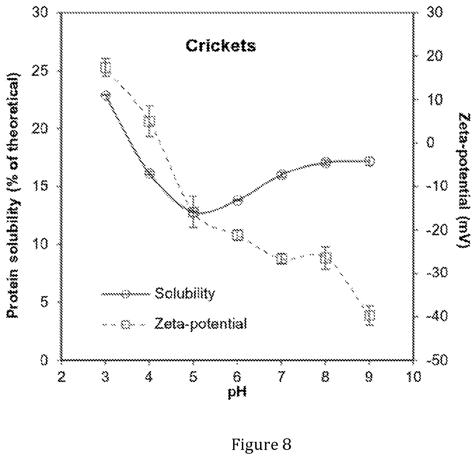

Proteins from both insects showed low solubility in water, ranging from 13 to 23% with crickets and 14 to 27% with mealworms (Figure 8). Proteins form both insects showed similar pH-dependent behaviour, exhibiting minimum solubility around pH 5 and maximum solubility at pH 3. Solubility at neutrality was 16 and 20% for crickets and mealworms, respectively, and increased slightly at alkaline pH.

Zeta-potential indicates the surface charge of particles. It is noted that the samples consisted of heterogeneous mixtures of proteins, and the zeta-potential of these samples is the sum of those proteins. Zeta-potentials of proteins from both insects were negative above ca. pH 4.3. The magnitudes of cricket proteins were slightly higher compared to mealworm proteins, e.g. 26 mV vs. 20 mV at pH 7.0. This can be related to differences in protein compositions or ionic strengths, which would shield protein charges and result in lower zeta-potential values. Although not analysed in the current study, crickets have been shown to contain more minerals than mealworms. The pH of zero zeta-potential generally overlaps with minimum solubility of the protein. It appears that minimum solubility occurs slightly above this pH in Figures 8, but tighter sampling intervals would be necessary to correctly identify the relationship between zeta-potential and solubility. The uneven shape of the curve of cricket proteins is likely to be caused by the presence of various proteins (Figure 8).

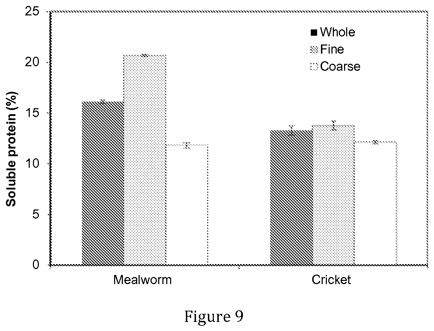

To further study the relevance of the enrichment of some proteins in fine and coarse fractions, the water solubility of defatted insect meals (obtained as described in Example 1) and each fraction (obtained as described in Example 2) were determined. Air classification influenced the protein water solubility of mealworm material: the fine fraction showed 28% elevated solubility (Figure 9). Some of the proteins enriched in this fraction (e.g. 75 and 77 kDa protein) may have higher solubility. In cricket material, the solubility of the coarse fraction was slightly lower than those of the defatted meal and the fine fraction. This may simply be caused by the larger particle size, as proteins may be trapped within larger structures and hence not extractable.

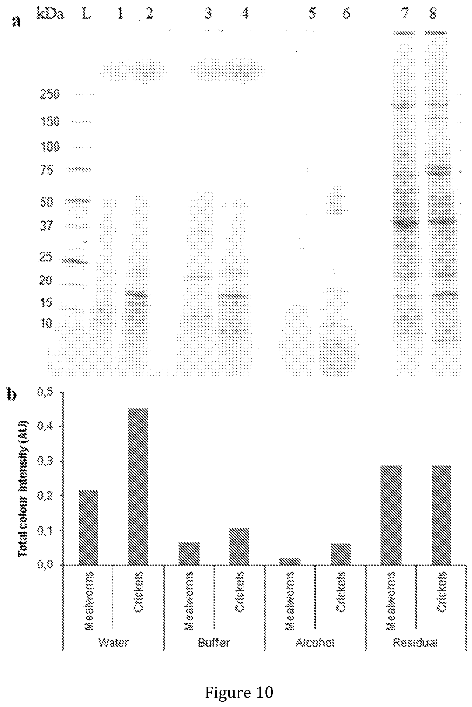

To gain information about the solubility of insect protein in different solvents, a modified Osborne extraction procedure was followed to separate proteins soluble in water, salt, alcohol and SDS/DTT and analysed electrophoretically (Figure 10). To this end, samples were sequentially extracted in the solvents (twice) and the soluble material was separated by centrifugation (10 000 g x 10 min). The pellet was suspended in the following solvents: (A) Water; (B) 0.5 M NaCl; (C) 70% ethanol; (D) 2% SDS, 6 M urea, 0.1 M dithiothreitol (DTT). The extracts varied in protein contents and were diluted in the following extract-to-water ratios before SDS-PAGE: A. 1:3; B. 7:1; C. 1:3; D. 1:0 (as is). Ethanol extracts (C) had to be diluted to enable sample loading, despite very low protein quantities. The loading volume was tripled for these samples. Electrophoresis was carried out as described.

Because of the interference of buffer components with protein determination assays, proteins were not quantified. Loading concentrations that give similar band intensities had to be determined empirically by running test gels with samples at varying concentrations. For rough quantification of the proteins extracted in different solvents, the band intensities were determined by image analysis and arbitrary quantities were calculated from these data corrected with dilution factors (Figure 10, lower part). On intensity basis, slightly below 50% of the extracted proteins appeared to be in the water extracts and the rest was roughly equally distributed between the rest of the solvents. Water extracts (lanes 1-2; Figure 10, upper part) contained mainly proteins with relative molecular weights below 25 kDa. The lanes visible in salt extractable proteins (lanes 3-4) were very similar to those of water extracts especially in the case of cricket proteins. Alcohol soluble proteins could be found in low quantities only in crickets (lane 6). The samples in the last fraction extractable in SDS, urea and DTT contained a large number of bands ranging from low (<10 kDa) to high molecular weights (>200 kDa) and very large protein aggregates that remained in the wells and could not penetrate the gel.

### Example 6. Sensory profiling

Sensory evaluation of the insect meals (crickets and mealworms in fine and coarse fractions obtained as described in Example 2) was carried out at the sensory laboratory of VTT, which fulfils the requirements of the ISO standards (ISO 2005 and 2007). The sensory panel consisted of 10 trained assessors. The method in sensory profiling was descriptive analysis (Lawless & Heymann, 2010), the evaluated attributes being coarseness of mouthfeel, saltiness, intensity of meat-like flavour, and flavour intensity. The attribute intensities (0 - 10) were rated on continuous graphical intensity scales, verbally anchored from both ends, where 0 = attribute not existing, 10 = attribute very clear. The samples were served to the assessors coded with three-digit numbers in random order in two replicates (crickets and mealworms in separate sessions, each sample twice in one session). The scores were recorded and collected using a computerized Compusense Five data system, Ver. 5.4 (Compusense, Guelph, Canada). Sensory profiling data were subjected to analysis of variance using IBM SPSS Statistics, Ver. 22 (IBM Corporation, New York, USA), and significant differences (p < 0.05) between individual means were identified by Tukey's test. Correlation coefficients (r) were analysed by Person's correlation with a 95% confidence level.

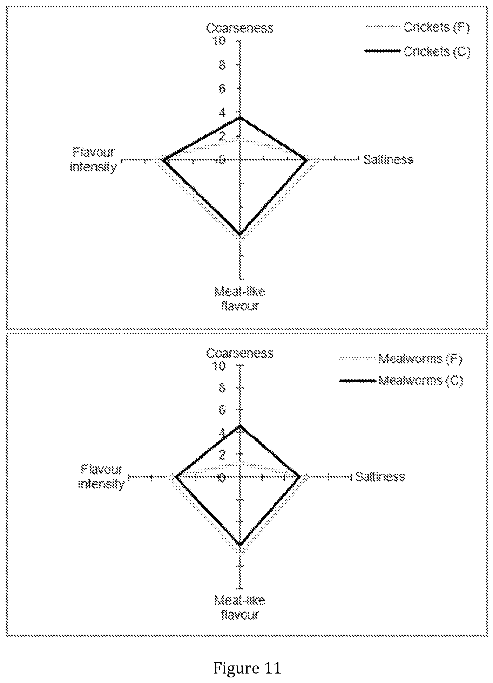

The fine and coarse cricket fractions deviated statistically significantly from each other in coarseness (p=0.004), saltiness (p=0.056) and flavour intensity (p=0.028), the fine one being more intense in saltiness and flavour intensity but more powdery than the coarse fraction (Figure 11). The fine and coarse mealworm samples differed statistically significantly from each other in coarseness (p=0.000) and meat-like flavour (p=0.008), the fine one having more intense meat-like flavour but being more powdery than the coarse fraction (Figure 11). Thus, according to the sensory results it seems that the impact of the fractionation process (fine vs. coarse) is clear on texture (coarseness) but not concordant to the flavour characteristics of the insect fractions being dependent of the insect variety. Nevertheless, the fine fractions exhibited saltier and more intense meat-like flavour with stronger flavour intensity compared to those of the coarse fractions.

### References

Bullard, B., Leonard, K., 1996. Modular proteins of insect muscle. Adv. Biophys. 33, 211-221. doi:10.1016/0065-227X(96)81676-5
Finke, M.D., 2002. Complete nutrient composition of commercially raised invertebrates used as food for insectivores. Zoo Biol. 21, 269-285. doi:10.1002/zoo.10031
Finke, M.D., 2007. Estimate of chitin in raw whole insects. Zoo Biology 26:105-115
Lawless, H.T., Heymann, H., 2010. Sensory evaluation of food principles and practises, descriptive analysis, 2nd ed. Gaithersburg: Chapman & Hall/Aspen Publishers, Inc.
Nowak, V., Persijn, D., Rittenschober, D., Charrondiere, U.R., 2016. Review of food composition data for edible insects. Food Chem. 193, 39-46. doi:10.1016/j.foodchem.2014.10.114
Sipponen, M.H., Pihlajaniemi, V., Vainio, H., Palonen, E., Hokkanen, S., Vahvaselkä, M., Pastinen, O., Nyyssölä, A., Laakso, S., 2016. Integrating the opposites of biofuel production: absorption of short-chain alcohols into oleaginous yeast cells for butanol recovery and wet-extraction of microbial oil. Green Chem. 2775-2781. doi:10.1039/C5GC03008K
van Huis, A., 2013. Potential of Insects as Food and Feed in Assuring Food Security. Annu. Rev. Entomol. 58, 120928130709004. doi:10.1146/annurev-ento-120811-153704
van Straaten, M., Goulding, D., Kolmerer, B., Labeit, S., Clayton, J., Leonard, K., Bullard, B., 1999. Association of kettin with actin in the Z-disc of insect flight muscle. J. Mol. Biol. 285, 1549-62. doi:10.1006/jmbi.1998.2386
Vincent, J.F. V, Wegst, U.G.K., 2004. Design and mechanical properties of insect cuticle. Arthropod Struct. Dev. 33, 187-199. doi:10.1016/j.asd.2004.05.006
Yi, L., Lakemond, C.M.M., Sagis, L.M.C., Eisner-Schadler, V., Huis, A. Van, Boekel, M.A.J.S. Van, 2013. Extraction and characterisation of protein fractions from five insect species. Food Chem. 141, 3341-3348. doi:10.1016/j.foodchem.2013.05.115

## Claims

1. A method of preparing fine and coarse arthropod meals, **characterized by** comprising the steps of:
(a) extracting fat from arthropods, whereby a defatted arthropod fraction and one or more fat fractions are obtained,
(b) dry-milling the defatted arthropod fraction into arthropod meal having such a particle size that at least 90% of the particles are smaller than 120 µm, based on volume,
(c) dividing the thus obtained defatted arthropod fraction into a fine arthropod meal fraction and a coarse arthropod meal fraction by at least one operation selected from sieving and air classification, wherein said fine fraction has a fat content of less than 5% based on dry matter and an average particle size below 40 µm, preferably below 15 µm, based on volume, and wherein said coarse fractions has a fat content of less than 5% based on dry matter and an average particle size over 40 µm, based on volume,
wherein the average particle size is determined by laser diffraction according to the description.

2. The method according to claim 1, wherein the arthropod meal obtained as the fine fraction has one or more characteristics selected from the group consisting of:
a protein content of at least 40%, preferably at least 50%, more preferably at least 52%, based on amino acid determination, wherein cysteine represents at least 1.7%, preferably at least 1.9% of the total amino acids, lysine represents at least 6.2%, preferably at least 6.9% of the total amino acids, and methionine represents at least 1.4%, preferably at least 1.7% of the total amino acids;
a chitin content of less than 50%, preferably less than 42%, based on non-protein nitrogen;
a fat content of less than 2.5%, more preferably less than 1%, based on dry matter.

3. The method according to claim 1, wherein the arthropod meal obtained as the coarse fraction has one or more characteristics selected from the group consisting of:
a protein content of at least 45%, preferably at least 53%, more preferably at least 55%, based on amino acid determination;
a chitin content of less than 55%, preferably less than 47%, based on non-protein nitrogen;
a fat content of less than 2.5%, more preferably less than 1%, based on dry matter.

4. The method according to any one of claims 1 to 3, wherein step (a) is carried out by extracting arthropods with a fluid in a supercritical state.

5. The method according to claim 4, wherein the fluid used in supercritical extraction is carbon dioxide and/or a combination of carbon dioxide and a C₁-C₄ alcohol, preferably ethanol.

6. The method according to any one of claims 1 to 3, wherein step (a) is carried out by solvent extraction.

7. The method according to claim 6, wherein the solvent used in solvent extraction is selected from the group consisting of hydrocarbons, chlorinated hydrocarbons, and ethers.

8. The method according to any one of claims 1 to 7, wherein the fat content of the defatted arthropod fraction obtained from step (a) is between 3% and 5%, less than 2.5%, or less than 1%.

9. The method according to any one of claims 1 to 8, wherein in steps (b) to (c) the moisture of the arthropod fractions less than 10%, preferably less than 5%.

10. The method according to any one of claims 1 to 9, wherein said division in step (c) by using air classification, the rotation speed of the air classifier being about 20 to 100% of the maximum rotation speed of the classifier and the airflow being 40 to 100% of the maximum airflow of the classifier.

11. The method according to any one of claims 1 to 10, wherein said division in step (c) is performed by using sieving, the sieve aperture size being 20 to 250 µm, preferably 25 to 100 µm.

12. Use of a fine or coarse arthropod meal obtainable by the method according to any one of claims 1 to 11 in foodstuffs, the pharmaceutical industry and cosmetics, wherein both fine and coarse arthropod meals have a fat content of less than 5% based on dry matter.

## Patentansprüche

1. Verfahren zur Herstellung feiner und grober Arthropodenmahlzeiten, **dadurch gekennzeichnet, dass** es die folgenden Schritte umfasst:
(a) Gewinnen von Fett von Arthropoden, wodurch eine entfettete Arthropodenfraktion und eine oder mehrere Fettfraktionen erhalten werden,
(b) Trockenmahlen der entfetteten Arthropodenfraktion in eine Arthropodenmahlzeit, die eine Partikelgröße aufweist, die derart ist, dass mindestens 90 % der Partikel, bezogen auf das Volumen, kleiner als 120 µm sind,
(c) Teilen der so erhaltenen entfetteten Arthropodenfraktion in eine feine Arthropodenmahlzeitfraktion und eine grobe Arthropodenmahlzeitfraktion mittels mindestens eines Vorgangs, der ausgewählt ist aus Sieben und Luftsichtung, wobei die feine Fraktion, bezogen auf die Trockenmasse, einen Fettgehalt von kleiner als 5 % und, bezogen auf das Volumen, eine durchschnittliche Partikelgröße von unter 40 µm, vorzugsweise unter 15 µm, aufweist, und wobei die grobe Fraktion, bezogen auf die Trockenmasse, einen Fettgehalt von weniger als 5 % und, bezogen auf das Volumen, eine durchschnittliche Partikelgröße von unter 40 µm aufweist,
wobei die durchschnittliche Partikelgröße mittels Laserdiffraktion gemäß der Beschreibung bestimmt wird.

2. Verfahren nach Anspruch 1, wobei die Arthropodenmahlzeit, die als die feine Fraktion erhalten wird, eine oder mehrere Eigenschaften aufweist, die ausgewählt werden aus der Gruppe, die besteht aus:
einem Proteingehalt, bezogen auf Aminosäurebestimmung, von mindestens 40 %, vorzugsweise mindestens 50 %, mehr zu bevorzugen mindestens 52 %, wobei Cystein mindestens 1,7 %, vorzugsweise mindestens 1,9 %, der Gesamtaminosäuren ausmacht, Lysin mindestens 6,2 %, vorzugsweise mindestens 6,9 %, der Gesamtaminosäuren ausmacht, und Methionin mindestens 1,4 %, vorzugsweise mindestens 1,7 %, der Gesamtaminosäuren ausmacht;
einem Chitingehalt, bezogen auf Nicht-Protein-Stickstoff, von weniger als 50 %, vorzugsweise weniger als 42 %;
einem Fettgehalt, bezogen auf die Trockenmasse, von weniger als 2,5 %, bevorzugter weniger als 1 %.

3. Verfahren nach Anspruch 1, wobei die Arthropodenmahlzeit, die als die grobe Fraktion erhalten wird, eine oder mehrere Eigenschaften aufweist, die ausgewählt werden aus der Gruppe, die besteht aus:
einem Proteingehalt, bezogen auf Aminosäurebestimmung, von mindestens 45 %, vorzugsweise mindestens 53 %, bevorzugter 55 %;
einem Chitingehalt, bezogen auf Nicht-Protein-Stickstoff, von weniger als 55 %, vorzugsweise weniger als 47 %;
einem Fettgehalt, bezogen auf die Trockenmasse, von weniger als 2,5 %, vorzugsweise weniger als 1 %.

4. Verfahren nach einem der Ansprüche 1 bis 3, wobei der Schritt (a) mittels Gewinnens von Arthropoden mit einem Fluid in einem überkritischen Zustand durchgeführt wird.

5. Verfahren nach Anspruch 4, wobei das Fluid, das in der überkritischen Gewinnung verwendet wird, Kohlendioxid und/oder eine Kombination von Kohlendioxid und einem C₁-C₄-Alkohol, vorzugsweise Ethanol, ist.

6. Verfahren nach einem der Ansprüche 1 bis 3, wobei Schritt (a) mittels Lösungsmittelgewinnung durchgeführt wird.

7. Verfahren nach Anspruch 6, wobei das bei der Lösungsmittelgewinnung verwendete Lösungsmittel ausgewählt wird aus der Gruppe, die besteht aus Kohlenwasserstoffen, chlorierten Kohlenwasserstoffen und Ethern.

8. Verfahren nach einem der Ansprüche 1 bis 7, wobei der Fettgehalt der im Schritt (a) erhaltenen entfetteten Arthropodenfraktion zwischen 3 % und 5 %, weniger als 2,5 % oder weniger als 1 % beträgt.

9. Verfahren nach einem der Ansprüche 1 bis 8, wobei in den Schritten (b) bis (c) der Feuchtegehalt der Arthropodenfraktionen weniger als 10 %, vorzugsweise weniger als 5 %, beträgt.

10. Verfahren nach einem der Ansprüche 1 bis 9, wobei bei dem Teilen in Schritt (c) unter Verwendung von Luftsichtung die Drehzahl des Luftsichters etwa 20 bis 100 % der maximalen Drehzahl des Sichters beträgt und die Luftströmung 40 bis 100 % der maximalen Luftströmung des Sichters beträgt.

11. Verfahren nach einem der Ansprüche 1 bis 10, wobei das Teilen in Schritt (c) unter Verwendung von Sieben durchgeführt wird, wobei die Sieböffnungsgröße 20 bis 250 µm, vorzugsweise 25 bis 100 µm, beträgt.

12. Verwendung einer feinen oder groben Arthropodenmahlzeit, die mittels des Verfahrens nach einem der Ansprüche 1 bis 11 erhalten werden kann, in Lebensmitteln, der pharmazeutischen Industrie und Kosmetik, wobei sowohl feine als auch grobe Arthropodenmahlzeiten einen Fettgehalt, bezogen auf die Trockenmasse, von weniger als 5 % aufweisen.

## Revendications

1. Procédé pour préparer des farines d'arthropodes fines et grossières, **caractérisé en ce qu'**il comprend les étapes de :
(a) extraction des matières grasses des arthropodes, moyennant quoi une fraction d'arthropodes dégraissée et une ou plusieurs fractions de matières grasses sont obtenues,
(b) broyage à sec de la fraction d'arthropodes dégraissées en farine d'arthropodes ayant une granulométrie telle qu'au moins 90 % des particules soient plus petites que 120 µm, sur la base du volume,
(c) division de la fraction d'arthropodes dégraissée ainsi obtenue en une fraction de farine d'arthropodes fine et une fraction de farine d'arthropodes grossière par au moins une opération choisie parmi un tamisage et une turboséparation, dans laquelle ladite fraction fine a une teneur en matières grasses inférieure à 5 % sur la base des matières sèches et une granulométrie moyenne inférieure à 40 µm, de préférence inférieure à 15 µm, sur la base du volume, et dans laquelle lesdites fractions grossières ont une teneur en matières grasses inférieure à 5 % sur la base des matières sèches et une granulométrie moyenne supérieure à 40 µm, sur la base du volume,
dans lequel la granulométrie moyenne est déterminée par diffraction de laser conformément à la description.

2. Procédé selon la revendication 1, dans lequel la farine d'arthropodes obtenues en tant que fraction fine a une ou plusieurs caractéristiques choisies dans le groupe constitué par :
une teneur en protéines d'au moins 40 %, de préférence d'au moins 50 %, mieux encore d'au moins 52 %, sur la base de la détermination des acides aminés, dans laquelle la cystéine représente au moins 1,7 %, de préférence au moins 1,9 % des acides aminés totaux, la lysine représente au moins 6,2 %, de préférence au moins 6,9 % des acides aminés totaux, et la méthionine représente au moins 1,4 %, de préférence au moins 1,7 % des acides aminés totaux ;
une teneur en chitine inférieure à 50 %, de préférence inférieure à 42 %, sur la base de l'azote non protéique ;
une teneur en matières grasses inférieure à 2,5 %, mieux encore inférieure à 1 %, sur la base des matières sèches.

3. Procédé selon la revendication 1,
dans lequel la farine d'arthropodes obtenues en tant que fraction grossière a une ou plusieurs caractéristiques choisies dans le groupe constitué par :
une teneur en protéines d'au moins 45 %, de préférence d'au moins 53 %, mieux encore d'au moins 55 %, sur la base de la détermination des acides aminés ;
une teneur en chitine inférieure à 55 %, de préférence inférieure à 47 %, sur la base de l'azote non protéique ;
une teneur en matières grasses inférieure à 2,5 %, mieux encore inférieure à 1 %, sur la base des matières sèches.

4. Procédé selon l'une quelconque des revendications 1 à 3, dans lequel l'étape (a) est effectuée par extraction d'arthropodes avec un fluide dans un état supercritique.

5. Procédé selon la revendication 4, dans lequel le fluide utilisé dans l'extraction supercritique est le dioxyde de carbone et/ou une combinaison de dioxyde de carbone et d'un alcool en C₁ à C₄, de préférence l'éthanol.

6. Procédé selon l'une quelconque des revendications 1 à 3, dans lequel l'étape (a) est effectuée par extraction au solvant.

7. Procédé selon la revendication 6, dans lequel le solvant utilisé dans l'extraction au solvant est choisi dans le groupe constitué par les hydrocarbures, les hydrocarbures chlorés, et les éthers.

8. Procédé selon l'une quelconque des revendications 1 à 7, dans lequel la teneur en matières grasses de la fraction d'arthropodes dégraissée obtenue dans l'étape (a) est comprise entre 3 % et 5 %, est inférieure à 2,5 %, ou est inférieure à 1 %.

9. Procédé selon l'une quelconque des revendications 1 à 8, dans lequel, dans l'étapes (b) à (c), l'humidité des fractions d'arthropodes est inférieure à 10 %, de préférence inférieure à 5 %.

10. Procédé selon l'une quelconque des revendications 1 à 9, dans lequel, dans ladite division dans l'étape (c) utilisant une turboséparation, la vitesse de rotation du turboséparateur est d'environ 20 à 100 % de la vitesse de rotation maximale du séparateur, et l'écoulement d'air est de 40 à 100 % de l'écoulement d'air maximal du séparateur.

11. Procédé selon l'une quelconque des revendications 1 à 10, dans lequel ladite division dans l'étape (c) est effectuée par utilisation d'un tamisage, la taille des ouvertures du tamis étant de 20 à 250 µm, de préférence de 25 à 100 µm.

12. Utilisation d'une faine d'arthropodes fine ou grossière pouvant être obtenue par le procédé selon l'une quelconque des revendications 1 à 11 dans des denrées alimentaires, dans l'industrie pharmaceutique, et dans des cosmétiques, dans laquelle les farines d'arthropodes tant fine que grossière ont une teneur en matières grasses inférieure à 5 % sur la base des matières sèches.
